# EUROPEAN PATENT APPLICATION

(11) **EP 0 731 179 A2**
(43) Date of publication of application: **11.09.1996**
(21) Application number: 96830094.7
(22) Date of filing: 06.03.1996
(51) Int. Cl.: C12Q 1/68, G01N 33/52, G01N 33/58, G01N 33/483, G01N 15/00, C12M 1/34

(54) **A method for carrying out a laboratory test of the in vitro integrity of haematic cells with no fixation and/or permeabilization, by DNA and general nucleic acid labeling and by flow cytometry reading**

(30) Priority: 09.03.1995 IT RM950142
(71) Applicant: Petraroli, Salvatore, 40135 Bologna (IT)
(72) Inventor: Petraroli, Salvatore, 40135 Bologna (IT)
(74) Representative: Sarpi, Maurizio

(57) **Abstract**

A method for carrying out a laboratory test of apoptosis of haematic cells *in vitro* with no fixation/permeabilization procedures, said method comprising the seeding of purified leukocytes, prepared by any way, within a test tube; the dyeing of DNA and of the nucleic acids in general; cold washing in an isotonic solution; suspension in a solution suitable for performing the reading of results by flow cytofluorometry. Reproducible results have been obtained even though the cells, before dyeing the DNA and the nucleic acids in general, are fixed by means of solutions containing typically paraformaldehyde in order to inactivate any pathogens possibly present.

## Description

This invention relates to a method for carrying out a laboratory *in vitro* examination of apoptosis on haematic cells with no fixation and/or no permeabilization process, by means of a DNA and general nucleic acids labeling compound which is excitable in the visible range, with reading performed by flow cytofluorometry.

Such method measures very precocious events of the apoptotic process, so that it allows a reliable response to be obtained in time ranges well shorter than those required by the methods already known, and in addition, owing to its simplicity of realization and interpretation as well as to the reproducibility of its results, it is especially suitable for clinical use. Advantageously, the process of cell fixation by means of solutions that typically contain paraformaldehyde does not affect the good quality of the results obtained.

The in *vitro* estimate of apoptosis is a technique that is employed at the present time in clinical and research laboratories in order to determine the tendency of cells to a programmed death in subjects suffering from HIV immunodeficiency correlated syndromes in subjects suffering from neoplasias, especially those of haematologic nature, who are subjected to cytotoxic treatments, and in subjects who suffer from autoimmune illnesses. The *in vitro* apoptosis in cells corresponds to what happens in vivo when apoptosizing active agents act on the cells.

After the contact with apoptosis-stimulating agents a series of complex metabolic events occur that end in the total fragmentation of DNA and then of the cell.

The apoptosis-stimulating agents employed in the tests carried out *in vitro* are innumerable and they vary according to the cell type. In many cases the apoptosis-inducing stimulus consists in the lacking of growth-suitable stimuli.

The observation that the metabolic events mentioned above occur in the apoptosis process, and the availability in the preceding years of a relatively low number of methods for estimating this phenomenon, have induced up to now the operators to employ the relatively late alterations of DNA instead of more precocious events as the apoptosis labeling agents.

The most recent methods employed in the study of apoptosis by putting into evidence the DNA fragmentation make use of molecular biology techniques that estimate the particular fragmentation mode of DNA which is typical of apoptosis, or they make use of fluorescent dyes which are specific for DNA or nucleic acids in general, and are analyzable through fluorescence microscopy or by means of flow cytofluorometry after fixation/permeabilization of cells and excitation in the visible range, or cells which have not been fixed/permeabilized and UV excitation.

In procedures that make use of evidencing of DNA fragmen-tation, the observation of a peculiar aspect of fragmentation is an index of apoptosis. The result obtained by such a method, however, can be affected by numerous variables that are connected to the sensitivity of the test, such variables including the percentage of cells in the apoptosis in a give sample, the lack of an accurate quantification of the phenomenon, the impossi-bility of estimating apoptosis in intact cells.

In procedures that make use of fluorescence microscopy, just a very low number of cells can be analyzed and anyway a subjectivity margin exists in the analysis itself; in procedures that make use of flow cytofluorometry and of DNA dyes, or of nucleic acids dyes in general, which can be excited in the visible range, cells are to be fixed/permeabilized so that a waste of time occurs and anyway the precocious phases of the apoptotic process are impossible to visualize.

Finally, the employment of permeating dyes and of intact cells has been limited up to now to dyes that are excitable in the UV, which is a technical difficulty insuperable for most laboratories, as the apparatuses suitable for detecting that type of excitation are extremely costly and complex.

In the course of the years the point has been reached of arranging and using systems for the estimate of apoptosis for clinical use by means of flow cytofluorometry, so that the rapid and reproducible quantitative determination of the phenomenon has been achieved. However, such procedures, if performed on intact cells, the only methods that allow the precocious phases of the apoptotic process with no need for particular preparation of cell cultures before they can be carried out, do not belong in the possibilities of the most part of laboratories, as they need DNA dyes that are excitable in the UV. This is the reason why such methods have spread almost exclusively throuout the most advanced research laboratories.

The object of the present finding is that of overcoming the drawbacks and the limitations as above and of suggesting a flow cytofluorometric laboratory test of new conception, whose assumption for the study of apoptosis is not the estimate of DNA fragmentation on fixed and/or permeabilized cells (though the test itself might supply results even on fixed and/or permeabilized cells) employing nucleic acids dyes that can be excited in the visible range or in the UV, but which allows a dye of the nucleic acids to be employed as an apoptosis labeler, capable of revealing the most precocious phases of the apoptotic process and can be employed on intact cells so shortening the incubation timees of cells and therefore the test times.

According to this invention, a laboratory test is provided for the estimate of *in vitro* apoptosis, on the basis of the biological principle that cells which are going to be apoptotic increase the cell DNA ability to be dyed with dyeing agents capable to permeate the cell membrane which is evidently not yet altered and capable of being excited in the visible range. Indeed, it is well known tha during the precocious phases of apoptosis, the permeability of the cell membrane and the accessibility of the specific dyeing agents to DNA and nucleic acids in general is progressively increased.

The procedure for carrying out a laboratory test of apoptosis in vitro according to this invention thus provides the labeling of DNA and, in general, of nucleic acids on intact cells, and the reading of results by flow cytometry with excitation in the visible range.

The labeling of DNA and of the nuclei acids in general is carried out preferably by dyeing with a dyeing solution containing a fluorescent labeling agent. Advantageously, before dyeing DNA and the nucleic acids in general, the labeling of cells of interest can be performed by means of fluorescent probes which are specific for cell structures so as to obtain not only information about the global ability of cells to enter apoptosis, but also the response ability to the stimulus, such ability being decomposed for the various sub-populations.

Accordingly, it is an object of this invention a method that employs a DNA dye, and in general a dyeing agent for nucleic acids in general, said method being capable of permeating the cell membrane of cells with no fixation and/or permeabilization process, but the agent being anyway excitable in the visible range for the realization of a laboratory test of apoptosis *in vitro* after the preparation of the cells of interest which can be performed in any way.

More specifically, the procedure for the realization of a laboratory test of apoptosis in vitro according to the present finding, after the usual stages of: drawing venous blood in the presence of an anti-coagulant agent; and optionally of: pre-purification of cell populations, resuspension of the same in standard cultural soil and partitioning into at least two test-tubes, addition of apoptotic stimuli in at least one of said test-tubes; is characterized in that it comprises the following phases:
lysis of the erythrocytes by means of a solution of a lysis agent so that the viability of leucocytes is kept unaltered;
washing in an isotonic solution;
dyeing of DNA and of the nucleic acids in general, by means of a fluorescent labeling compound capable of permeating the cell membrane;
suspension in a solution suitable for performing the reading of flow cytofluorometry results.

In the case of samples made up of purified mononucleates, before the lysis of erythrocytes an optional incubation phase is provided at a temperature compatible with the occurrence of the optotic process for a time which is necessary and sufficient for the process itself to occur, in the presence of a specific culture soil.

Again optionally, before dyeing DNA and the nucleic acids in general, the labeling of the cells of interest can be carried out with fluorescent probes having emission spectra in the green band (about 530 nm) and in the orange band (about 570 nm), for instance CD4, CD8, etc., in order to estimate the different dyeing possibilities of DNA in the various lymphocytary subgroups.

It has been observed that apoptosis can be well put into evidence both in cells as just drawn, for instance from HIV+ patients, or for instance in irradiated mononucleates from healthy subjects already after six hours.

It has been advantageously found that in order to put into evidence the apoptosis in HIV+ subjects it is not necessary to perform any incubation period and any purification of the mononucleates. This allows most precocious phases of the apoptotic process to be put into evidence, and a lot of time to be saved.

In case pre-purified cell preparations and incubation in culture soil are optionally employed, as already discussed above, the addition of apoptotic stimuli occurs with the following modalities:
a) no stimulus, i.e. just the culture soil is employed as the carrier of the apoptosizing compounds, in order to obtain the control of spontaneous apoptosis;
b) one or more apoptosizing stimuli among all the very numerous ones that exist in relationship to the type of cells in the culture.

The stimulus-inducing agent can also be diluted in a suitable way if one thinks that it is necessary to estimate the response to different concentrations of the stimulating compound.

The preparation of the diagnostic test then provides: a test-tube containing whole lysed blood for the immediate test, or if some culture are to be examined, one or more test-tubes not containing any stimulus in order to obtain the control of spontaneous apoptosis of lymphocytes, and one or more test-tubes containing apoptosis stimuli.

The incubation of cells so treated occurs at a temperature that allows the apoptotic process to occur for a period that depends on the stimulating compound employed and on its amount.

Just for orientative purposes, it is mentioned here that such period is within 24 and 72 hours.

The incubation can be carried out in any kind of thermostatic device, oven, steam-bath, cell culture incubator, on condition that the stability of temperature and the pH value that allow the cell metabolism are warranted.

The incubation solution can be any sterile isotonic solution at a pH value compatible with the apoptotic process.

After the selective lysis of the red cells, which is not necessary in the case of use of purified leukocytes cultures, and after a washing with an isotonic solution, the DNA and general nucleic acids dyeing is carried out.

However, before dyeing DNA it is possible to perform both the labeling of cells with specific probes for lymphocytic sub-poplations, and the fixation of samples by means of fixative solutions for flow cytometry, said solutions containig typically 0.4 - 1% paraformaldehyde.

According to a preferred embodiment of this invention, the dyeing of DNA and of nucleic acids in general, is carried out by incubating cells in a solution containing the fluorescent labeling compound, having the chemical name 6-dimethylamino-2-[4-[4-(dimethylamino)phenyl]-1,3-butadienyl]-1-ethyl quinolinium perchlorate at a temperature that allows the dyeing agent to get into the cells. The concentration is in the range from 100 ng/ml and 100 mg/ml.

In any case, any other dyeing agent for the DNA and the nucleic acids in general can be employed, such agent having the same characteristics.

The washing and resuspending solution for the reading of results with the cytometre can be any saline solution, even not a sterile one, which is isotonic and at a pH compatible with the cell viability and not interfering with the emission of the fluorescent tracers.

The events lying in the classical lymphocytic and/or monocytic and/or granulocytic region are analyzed by the cytometre, if a whole blood is under test after erythrocyte lysis (minimum of 5,000 events per each population). The result of the test is expressed as the percentage of cells that accept the dyeing agent.

If cell sub-populations have been labeled, the calculation is performed in a distinct way on the sub-populations themselves.

The advantages of the method which is the object of this invention with respect to the procedures known are evident from the preceding discussion:
the test can be carried out immediately, on a blood sample that has been just drawn, and in very short operation times;
the test analyses intact cells, and it does not require complicated fixation/permeabilization procedures, which thinghas been possible up to now by means of the use of dyeing agents ehich are excitable in the UV and whose employment is thus limited to very few laboratories;
it is very easy to read, as it asks just for procedures which are similar to the standard ones for immunofluorescence;
fixation of cells can be realized in a typical way using 0.4 - 1% paraformaldehyde, with no damage to the clinical test result;
it supplies useful indications not only as to the amount of apoptosic cells, but also as to the most sensitive or apoptosis-resistant cells. Such a feature can be usefully applied in some specific pathologies.

The specific practical work example shown below allows such and further advantages of this invention to be fully appreciated.

### EXAMPLE

The commercial configuration for realizing the test according to this invention essentially includes:
- a solution for carrying out the selective lysis of blood red cells
- a dyeing solution
   and optionally
- an isotonic washing solution.

The technical procedure for performing the test is as follows:
1) Venous blood is drawn. 0.5 ml of blood are put into the test tube.
2) The solution, based on NH₄Cl buffered at pH 7.2 and at equilibrium temperature for carrying out the lysis process is added. The test tube content is stirred by a Pasteur-type pipette 3 - 5 times.
3) The solution for carrying out the lysis process is allowed to act for 10 - 15 minutes at room temperature.
4) The test tube is centrifuged at 350 x g for 8 minutes, then the supernatant is sucked off and the pellet is resuspended in 0.5 ml of isotonic solution.
5) 500 ml of the solution of a labeling compound like that commercially available with the name LDS751 at 1mg/ml previously prepared in an isotonic solution. Starting from this moment and for the subsequent operations, it is advisable to avoid direct exposure of samples to light.
6) The test tube is shaken gently and left in the incubator for 10 - 15 minutes at room temperature.
7) The analysis by the flow cytometre is performed according to the standard routine methods used for immunofluorescence, such as the determination of the amplifications and of the proper electronic thresholds for the measurement of the fluorescence generated by the LDS751 in the channel of the orange color (about 570 nm) or, if available, preferably of the red band (> 650 nm).
8) The amount of apoptotic cells is evaluated as the number of cells whose fluorescence lies beyond a maximum value set forth by employing a healthy sample as the control sample.
9) Optionally, it is possible to perform the determination of apoptotic haematic cells also on purified populations, e.g. of mononucleates obtained by means of one of the numerous standard procedures routinely employed in the laboratories. In that case, the steps 1 - 4 are omitted. The steps 5 - 8 are kept unchanged.

A preferred embodiment of this finding has been disclosed herein. However, it is evident that numerous variants and modifications can be introduced by those who are skilled in the art without going outside the scope and spirit of the invention as it is defined in the claims enclosed herein below.

## Claims

1. A method for carrying out a laboratory test of apoptosis of haematic cells *in vitro*, comprising the seeding of purified leukocytes prepared in any way within a test tube, said method being characterized in that it comprises the following operations in combination:
washing in an isotonic solution;
dyeing of DNA and of the nucleic acids in general, by means of a labeling compound which is capable of permeating the cell membrane that is unaltered as yet, without asking for fixation/permeabilization procedures, and which is excitable in the visible band; said labeling compound being anyway employable also on cells that have been fixed by means of paraformaldehyde containing solutions;
suspension into a solution that is suitable for performing the reading of results by flow cytometry.

2. A method according to the preceding claim , characterized in that it further includes, before carrying out the dyeing of DNA and of the nucleic acids in general, a labeling for the cells of interest with cell anti-subpopulation probes to evaluate the different nuclear fluorescence in the different cell subgroups.

3. A method according to the preceding claims, wherein no previous incubation of the haematic cells is necessary as soon as they are drawn, before performing the test and no fixation/per-meabilization procedure.

4. A method according to the preceding claims, wherein the labeling compound is a fluorescent labeling compound having the chemical name 6-dimethylamino-2-[4-[4-(dimethylamino)phenyl]-1,3-butadienyl]-1-ethyl quinolinium perchlorate

5. A method according to the preceding claims, wherein the dyeing of DNA and of the nucleic acids in general is carried out by incubating the cells in a dyeing solution that contains said labeling compound for 8 - 30 minutes at room temperature.

6. A method according to the preceding claims, wherein the dyeing solution contains LDS751 at a concentration between 100 ng/ml and 100 mg/ml.

7. A method according to the preceding claims, wherein after the labeling operation carried out with fluorescent probes specific for cell structures (CD4, CD8 and similar ones), a resuspension into an isotonic solution suitable for the flow cytofluorometry is provided.

8. A method according to the preceding claims, wherein the washing and resuspension solution for performing the reading of results by the cytometre is any saline solution, even a non-sterile one, that is isotonic and at a pH value compatible withthe cell viability, and that does not interfere with the emission of the fluorescent tracers.

9. A method according to the preceding claims, for carrying out a laboratory test of apoptosis in vitro by labeling DNA and nucleic acids in general, and by reading results through flow cytofluorometry substantially as disclosed, exemplified and claimed above.
